(19) Europäisches Patentamt
European Patent Office
Office européen des brevets

(11) Veröffentlichungsnummer: **0 069 379**
**B1**

(12) # EUROPÄISCHE PATENTSCHRIFT

(45) Veröffentlichungstag der Patentschrift: **24.06.87**

(51) Int. Cl.⁴: **C 12 N 11/06**, C 12 N 11/08, C 12 N 11/10, C 12 Q 1/62

(21) Anmeldenummer: **82105993.8**

(22) Anmeldetag: **05.07.82**

(54) **Lösliche Leber-Uricase, Verfahren zu ihrer Herstellung und Verwendung.**

(30) Priorität: **07.07.81 DE 3126759**

(43) Veröffentlichungstag der Anmeldung: **12.01.83 Patentblatt 83/2**

(45) Bekanntmachung des Hinweises auf die Patenterteilung: **24.06.87 Patentblatt 87/26**

(84) Benannte Vertragsstaaten: **AT BE CH DE FR GB IT LI LU NL SE**

(56) Entgegenhaltungen:
**EP - A - 0 012 446**

**CHEMICAL ABSTRACTS, Band 89, 1978, Seite 255, Nr. 19197d, Columbus, Ohio, USA; M.H. REMY et al.: "Insolubilization and charge effects on crosslinked enzyme polymers. Kinetic studies in solution and gelified membranes"**
**BIOLOGICAL ABSTRACTS, Band 67, 1979, Nr. 15008; M. McCARTHY et al.: "Urate oxidase immobilization on elastin"**
**BIOLOGICAL ABSTRACTS, Band 68, 1979, Nr. 19742; P.V. SUNDARAM et al.: "Immobilized-enzyme nylon-tube reactor for routine determination of uric acid in serum"**
**CLINICAL CHEMISTRY, Band 25, Nr. 10, Oktober 1979, Seiten 1744-1748, Easton, Pennsylvania, USA; R.**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Gloger, Manfred, Dr., Karwendelstrasse 6, D-8120 Weilheim (DE)**
Erfinder: **Heinle, Josef, Bayrischzeller Strasse 29, D-8000 München 90 (DE)**
Erfinder: **Schlumberger, Helmut, Kaiser-Heinrich Strasse 23, D-8121 Polling (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

(56) Entgegenhaltungen: (Fortsetzung)
**CHIRILLO et al.: "The use of immobilized enzyme reactors in continuous-flow analyzers for the determination of glucose, urea and uric acid"**
**BIOLOGICAL ABSTRACTS, Band 73, 1982, Nr. 67870; A.B. SALLEH et al.: "Some kinetic studies on immobilized uricase"**

EP 0 069 379 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine auch bei niedrigen pH-Werten lösliche Leberuricase, ein Verfahren zu ihrer Herstellung und ihre Verwendung zur Bestimmung der Harnsäure.

Uricase ist ein weit verbreitet im Tierreich, insbesondere in der Leber von Tieren vorkommendes Enzym, welches die Oxidation von Harnsäure in Gegenwart von Sauerstoff unter Bildung von Allantoin, $H_2O_2$ und $Co_2$ katalysiert gemäss nachfolgender Reaktionsgleichung:

$$2\,H_2O + \text{Harnsäure} + O_2 \rightarrow \text{Allantoin} + H_2O_2 + CO_2.$$

Aufgrund der obigen Reaktion würde sich Leberuricase gut für die Bestimmung der Harnsäure eignen, indem eines der Reaktionsprodukte, insbesondere $H_2O_2$, nach hierfür üblichen Methoden bestimmt wird. Ein wesentlicher Nachteil der Leberuricase liegt jedoch darin, dass das Enzym nur bei relativ hohen pH-Werten, normalerweise zwischen pH 9 und 11, löslich ist. Es ist daher schwierig, die durch die Leberuricase katalysierte Reaktion mit weiteren Reaktionen zu koppeln wie sie beispielsweise für die enzymatische Bestimmung des gebildeten $H_2O_2$ erforderlich sind, da hierfür in der Regel etwa neutrale pH-Werte erforderlich sind.

Es sind zwar Uricasen bekannt, die auch bei neutralem pH-Wert ausreichend löslich sind, diese müssten jedoch aus Mikroorganismen wie Cancida utilis oder Aspergillus flavus gewonnen werden und sind daher wesentlich teurer was ihre Verwendbarkeit für analytische Zwecke stark beschränkt. Dies gilt auch für eine aus der EU-AS 0 012 446 bekannten saure Uricase aus Streptomyces.

Der Erfindung liegt daher die Aufgabe zugrunde, dass an sich leicht zugängliche Enzym aus Leber, welches beispielsweise aus Schweineleber bei pH-Werten über 9 herausgelöst werden kann, so zu verändern, dass die oben beschriebenen Nachteile beseitigt und das Enzym auch in neutralem oder saurem Medium eingesetzt werden kann.

Gelöst wird diese Aufgabe erfindungsgemäss durch eine Leberuricase, die dadurch gekennzeichnet ist, dass sie kovalent an ein wasserlösliches Polysaccharid, Polysäureanhydrid, Polyvinylpyrrolidon oder Säureanhydrid als Trägersubstanz gebunden vorliegt und noch bei pH-Werten unter 6 löslich ist.

Der Erfindung liegt die überraschende Tatsache zugrunde, dass eine Leberuricase, die an eine wasserlösliche Trägersubstanz der genannten Art gebunden wird, ohne wesentliche Veränderung ihrer sonstigen Eigenschaften nicht nur bei neutralen pH-Werten, sondern auch im sauren Bereich ausgezeichnet löslich ist. So zeichnen sich erfindungsgemässe Präparate sogar durch Löslichkeit bei pH-Werten zwischen 1 und 11 aus. Ausserdem stellte sich heraus, dass das erfindungsgemäss modifizierte Enzym bis herab zu pH 5,0 nicht nur löslich, sondern auch aktiv ist und beispielsweise bei pH 7,0 noch etwa 40% der im pH-Optimum von pH 9,5 gemessenen Aktivität aufweist. Es eignet sich damit ausgezeichnet zur Bestimmung der Harnsäure auch in neutralem Medium.

Native Leberuricase geht im allgemeinen erst bei pH-Werten von 9,5 und höher in Lösung und fällt bei pH-Werten unter 9,0 aus der Lösung wieder aus. In der erfindungsgemässen modifizierten Form bleibt jedoch die Löslichkeit bis herab zu pH 1 bestehen. Die erfindungsgemässe Leberuricase lässt sich aufgrund dieser Eigenschaft leicht von allen bekannten Uricasen unterscheiden. Bemerkenswert ist ausserdem, dass die erfindungsgemäss modifizierte Leberuricase ihre immunologischen Eigenschaften unverändert beibehält und daher auch in der trägergebundenen Form immunologisch als Leberuricase erkannt und von Uricase anderen Ursprungs unterschieden werden kann.

Es ist bekannt, dass die Eigenschaften von Enzymen durch Trägerfixierung verändert werden können. So ist eine Veränderung der Aktivität, Verschiebung des pH-Optimums, Veränderung der Michaelis-Konstante, der spezifischen Aktivität und der Stabilität von Enzymen beschrieben worden. Es war jedoch nicht bekannt, dass durch Bindung an lösliche Träger die Löslichkeitseigenschaften eines Enzyms so drastisch verändert werden können, wie dies beim erfindungsgemässen Produkt der Fall ist. Aus Enzyme 26, 49–53 (1981) ist es bekannt, eine Hefeuricase (as Candida utilis) an Polyäthylenglycol zu binden, wobei man ein lösliches Produkt erhält, welches hinsichtlich seiner immunologischen Eigenschaften untersucht wurde. Eine Veränderung der Löslichkeit wurde hierbei nicht berichtet.

Als lösliche Trägersubstanzen werden im Rahmen der Erfindung Polysaccharide bevorzugt. Besonders bevorzugt wird ein wasserlösliches Dextran. Sehr gute Eregbnisse wurden jedoch auch mit anderen löslichen Polysacchariden wie insbesondere löslicher Stärke, Zuckern oder synthetischen Zuckerpolymeren erzielt. Unter den Zukkern erwiesen sich wiederum die Saccharide wie Saccharose als besonders geeignet. Auch hochmolekulare Polymere von Mono- und Disacchariden, wie sie beispielsweise durch Umsetzung mit Epihalogenhydrin erhalten werden, erwiesen sich als gut geeignet und werden im Rahmen der Erfindung den Polysacchariden zugerechnet.

Die geeigneten Trägersubstanzen sind jedoch nicht auf Polysaccharide beschränkt und auch Säureanhydride, Polysäureanhydride oder Polyvinylpyrrolidon sind geeignet. Ein Beispiel für ein geeignetes Säureanhydrid ist Bernsteinsäureanhydrid, für ein Polyanhydrid ein Copolymer von Methylvinyläther mit Maleinsäureanhydrid. Auch Succinimidderivate erwiesen sich als geeignet.

Michaelis-Konstante $K_M$ und Maximalgeschwindigkeit $V_{max}$ sind bei dem erfindungsgemäss modifizierten Enzym etwas verändert gegenüber der nativen Leberuricase. Es hat sich gezeigt, dass diese Veränderung in gewissem Masse vom Molekulargewicht der Trägersubstanz abhängig ist. Dabei werden die $K_M$-Werte kaum beeinflusst, während die $V_{max}$ umgekehrt proportional zum Molekulargewicht des Trägers verringert wird. Insbesondere bei hochmolekularen Trägermaterialien wie Stärke und Dextran sollte jedoch ein Molekulargewicht von mindestens 1000 vorliegen.

Das Molekulargewicht der erfindungsgemäss modifizierten Leberuricase hängt ab vom Molekulargewicht des Trägermaterials. So wurde für mit Dextran T 10 modifiziertes Enzym ein Molekulargewicht zwischen 180 000 und 200 000 Dalton festgestellt, während bei Dextran T 40 das Molekulargewicht zwischen 350 000 und 500 000 Dalton lag. Die Molekulargewichtsbestimmung wurde durch Gelchromatographie in Citratpuffer pH 5,8 durchgeführt. Das Molekulargewicht der nativen Leberuricase lässt sich unter diesen Bedingungen wegen der Unlöslichkeit des Enzymes nicht bestimmen.

Michaelis-Konstante $K_M$ und Maximalgeschwindigkeit $V_{max}$ wurden für native Uricase und die beiden vorstehend erwähnten Dextrane als Trägermaterialien für das Enzym bestimmt. Die Ergebnisse waren wie folgt:

| | $K_M$ [M] | $V_{max}$ [U/mg Protein] |
|---|---|---|
| native Uricase | $3{,}17 \cdot 10^{-6}$ | 10,5 |
| Uricase/Dex. T10 | $5{,}0 \cdot 10^{-6}$ | 5,6 |
| Uricase/Dex. T40 | $3{,}17 \cdot 10^{-6}$ | 3,7 |

Die erfindungsgemäss modifizierte Leberuricase ist mit Ammoniumsulfat nicht mehr fällbar, wird jedoch mit Aceton gut gefällt. Die Acetonfällung ist unter Erhalt der Aktivität reversibel. Sie eignet sich daher insbesondere auch zur Isolierung des erfindungsgemässen Enzyms.

Die Temperaturstabilität des Enzyms bei höheren Temperaturen (60 °C) ist wesentlich besser als beim nativen Enzym. Während letzteres bei dieser Temperatur nach 200 Minuten nur noch 5% der Ausgangsaktivität aufweist, beträgt die Aktivität des erfindungsgemässen Enzyms zum gleichen Zeitpunkt noch 82%. Nach 240 Minuten bei 60 °C ist das native Enzym inaktiv, das erfindungsgemässe Enzym weist noch 79% der Ausgangsaktivität auf.

Die Herstellung des erfindungsgemässen Enzyms erfolgt in an sich bekannter Weise, indem man die Uricase in wässriger Lösung mit dem gelösten Trägermaterial, welches wenigstens eine in wässriger Lösung mit $NH_2$-Gruppen reaktionsfähige Gruppe enthält, bei pH-Werten zwischen 9 und 11 umsetzt.

Besonders gute Ergebnisse wurden mit Trägersubstanzen erzielt, welche mit Bromcyan oder Cyanurochlorid aktivierte OH-Gruppen tragen. Die Kupplung erfolgt zweckmässig bei Temperaturen zwischen 0 °C und Raumtemperatur, jedoch können auch höhere oder niedrigere Temperaturen angewendet werden. Bevorzugt erfolgt die Kupplung bei pH-Werten zwischen 10,0 und 11,0.

Jedoch können auch die anderen, dem Fachmann bekannten Methoden der Enzymfixierung angewendet werden.

Um eine möglichst hohe Aktivitätsausbeute zu erzielen, erwies es sich als vorteilhaft, die molaren Verhältnisse von Enzym zu Trägermolekülen so zu wählen, dass nur ein relativ kleiner Teil der $NH_2$-Gruppen des Enzyms modifiziert wird. Das gilt insbesondere bei niedermolekularen Trägermaterialien. Die jeweils geeigneten Mengenverhältnisse lassen sich durch Vorversuche mit unterschiedlicher Trägermenge anhand der jeweils erzielten Aktivitätsausbeuten leicht feststellen.

Nach Abschluss der Kupplungsreaktion kann das erfindungsgemässe Enzym beispielsweise durch Zusatz von Aceton ausgefällt und isoliert werden. Vorzugsweise wird die erhaltene Lösung jedoch lyophilisiert, zweckmässig nach vorheriger Reinigung durch Dialyse. Bei der Gefriertrocknung werden vorzugsweise Stabilisierungsmittel zugesetzt wie sie üblicherweise bei der Lyophilisierung von Enzymen verwendet werden. Besonders geeignet erwiesen sich als stabilisierende Zusätze für die Lyophilisierung Saccharose und Mannit, die allein oder gemeinsam eingesetzt werden können.

Erfindungsgemässe Enzympräparate eignen sich wie bereits erwähnt ausgezeichnet zur Bestimmung der Harnsäure. Ein hierfür geeignetes Reagenz gemäss der Erfindung besteht daher aus erfindungsgemässer löslicher Leberuricase, Puffer und einem System zur Bestimmung von $H_2O_2$, von Allantoin oder von $CO_2$. Auch ist es möglich, die Sauerstoffabnahme, beispielsweise mit einer Sauerstoffelektrode, als Mass für die vorhandenen Harnsäuremengen zu messen.

Die folgenden Beispiele erläutern die Erfindung weiter:

Beispiel 1

a) Vorbereitung des Enzyms

5 g Uricase (BM 150746, 30 bis 50 U/ml, 9 bis 10 U/mg Protein) werden bei 4 °C gegen 4 × 2 l Natriumcarbonat/Natriumbicarbonat-Puffer (0,1 M, pH 10,2) dialysiert. Es wird dabei eine klare Enzymlösung erhalten.

b) Aktivierung des Dextrans

Lösliche Dextrane der Firma Roth, Karlsruhe (mittlere Molekulargewichte 10 000, 20 000, 40 000 und 500 000) werden mit Bromcyan (Fa. Merck) bei pH 10,6 aktiviert.

Dazu werden 30 g Dextran in 3 l Wasser gelöst und mit 2 n Natronlauge auf pH 10,6 eingestellt. Unter Rühren werden insgesamt 9 bis 15 g Bromcyan in 3 g Portionen innerhalb von 40 min. in den Ansatz gegeben. Der pH-Wert wird dabei mit Hilfe eines Autotitrators durch Zugabe von 2 bis 5 n Natronlauge auf pH 10,6 gehalten. Die Aktivierungsreaktion ist beendet, wenn keine Natronlauge mehr verbraucht wird, was nach etwa 1,5 bis 2 Stunden der Fall ist.

Es liegt dann eine klare Lösung vor, der pH-Wert wird mit 2 n Salzsäure auf 10,0 eingestellt. Das so aktivierte Dextran wird innerhalb von 2 Stunden bei 20 °C ausreichend gegen Wasser dialysiert.

c) Bildung der Uricase

Die vorbereitete Enzymlösung (a) wird unter kräftigem Rühren mit der aktivierten Dextranlösung (b) vereinigt. Gesamtvolumen 3,1 bis 3,2 l, pH 10,1 bis 10,3. Bei 4 °C oder Raumtemperatur wird 16 Stunden lang leicht gerührt.

Nach dieser Zeit sind noch etwa 80% der eingesetzten Aktivität vorhanden. Der Ansatz sieht klar aus.

d) Aufreinigung der modifizierten Uricase

Die modifizierte Uricase wird anschliessend gegen 0,04 M Natrium-Citratpuffer, pH 5,8 (enthalten 0,01% Natriumacid) dialysiert. Dauer etwa 3 Stunden. Anschliessend werden 30 g Saccharose und 45 g Mannit zur Lösung gegeben, und man filtriert klar. Die Lösung wird dann lyophilisiert.

75% der eingesetzten Aktivität befinden sich im Lyophilisat.

Beispiele 2 bis 12

Das Verfahren von Beispiel 1 wurde wiederholt unter Abänderung der Bedingungen und Trägerstoffe. Die Bedingungen und Resultate zeigt nachstehende Tabelle:

| Beispiel | 2 | 3 | 4 | 5 | 6 |
|---|---|---|---|---|---|
| Uricase (mg-Protein) | 50 | 50 | 50 | 200 | 500 |
| Uricase (Akt. units) | 360 | 360 | 360 | 1.800 | 5.000 |
| Träger Dextran, Mol. Gew. | 40.000 | 40.000 | 40.000 | 40.000 | 40.000 |
| andere | – | – | – | – | – |
| Gew-Verhältnis Träger: BrCN | 2:1 | 2:1 | 2:1 | 2:1 | 2:1 |
| Gew-Verhältnis Träger: Protein | 2:1 | 4:1 | 8:1 | 4:1 | 6:1 |
| Dauer h | 16 | 16 | 16 | 3+13 | 16 |
| Temperatur °C | 4° | 4° | 4° | 35° 4° | 4° |
| Puffer f. Lyophilisat | Kalium-Phosphat, 0,07 M, pH 7,0 | | | K-Phos., 0,07 M, pH 7,0 | 0,04 M Citrat, pH 5,8 |
| Zusätze bei Lyophilisation | – | – | – | – | Saccharose, Mannit |
| Ausbeuten g Lyophilisat | 0,178 | 0,420 | 0,700 | 1,7 | – |
| Aktivitätsausbeuten % nach Fixierung | 91 | 85 | 68 | 82 | 81 |
| % nach Lyophilisation | – | – | – | 71 | 77 |
| Spez. Akt. (U/mg Lyophilisat) | 1,32 | 0,61 | 0,30 | 0,76 | 0,21 |
| Stabilität (Restaktivität nach 3 Wochen bei 35°C) | – | – | – | 33% | 100% |

| Beispiel | 7 | 8 | 9 |
|---|---|---|---|
| Uricase (mg-Protein) | 500 | 100 | 200 |
| Uricase (Akt. in units) | 5.000 | 720 | 1.800 |
| Träger Dextran, Mol. Gew. | 20.000 | – | – |
| andere | – | Ficoll 70[1] | Saccharose |
| Aktivierung Träger: BrCN | 2:1 | 2:1 | 2:1 |
| Bindung Träger: Protein | 6:1 | 3:1 | 4:1 |
| Dauer h | 16 | 16 | 16 |
| Temperatur °C | 4° | 4° | 4° |
| Puffer f. Lyophilisation | 0,04 M Citrat, pH 5,8 | Kalium-Phosphat-Puffer 0,07 M, pH 7,0 | |

[1] Ein Rohrsaccharose-Epichlorhydrin-Copolymer MG 70 000

| Beispiel | 7 | 8 | 9 |
|---|---|---|---|
| Zusätze bei Lyophilisation | Saccharose Mannit | – | – |
| Ausbeuten g Lyophilisation | – | 0,7 | 1,0 |
| Aktivitätsausbeuten | | | |
| % nach Fixierung | 70% | 85% | 43% |
| % nach Lyophilisat | – | – | 36% |
| Spez. Akt (U/mg Lyophilisat) | 0,21 | 0,73 | 0,68 |
| Stabilität (Restaktivität nach 3 Wochen bei 35°C) | 100% | – | 51% |

| Beispiel | 10 | 11 | 12 |
|---|---|---|---|
| Uricase (mg Protein) | 10.000 | 100 | 40.000 |
| Uricase (Akt. in units) | 100.000 | 900 | 40.000 |
| Träger, Dextran, Mol. Gew. | 40.000 | 40.000 | 40.000 |
| andere | – | – | – |
| Aktivierung Träger: BrCN | 2:1 | 5:1 | 2:1 |
| Bindung Träger: Protein | 4:1 | 6:1 | 6:1 |
| Dauer h | 16 | 16 | 16 |
| Temperatur °C | 4 | 4 | 25 |
| Puffer f. Lyophilisation | 0,04 M Citr. Puffer pH 4,8 | 0,04 M Citr. Puffer pH 5,8 | 0,04 M Citrat, pH 5,8 |
| Zusätze bei Lyophilisation | Raffinose + Mannit | Saccharose + Mannit | Saccharose + Mannit |
| Ausbeuten g Lyophilisat | 185 | – | 1.127 |
| Aktivitätsausbeuten | | | |
| % nach Fixierung | 82 | 90 | 80 |
| % nach Lyophilisation | 71,5 | – | 69 |
| Spez. Akt. (U/mg Lyophilisat) | 0,37 | – | 0,24 |
| Stabilität (Reaktivität nach 3 Wochen bei 35°C) | 92% | – | 100% |

Beispiel 13

5 ml Uricase in Glycerin ≙ 325 mg Enzymprotein werden in 45 ml Wasser gegeben, im Eisbad in Abständen von 15 Minuten 2 × 25 mg Bernsteinsäureanhydrid in fester Form zugegeben. Der pH-Wert wird bei 9,8 durch Zusatz von 0,5 n NaOH mittels Titrator konstant gehalten bis keine NaOH mehr verbraucht wird. Der Ansatz wird dann mit Wasser auf 250 ml verdünnt und weitere 27 mg Bernsteinsäureanhydrid werden zugegeben und bei pH 9,8 wird wie vorstehend weitertitriert bis zum Reaktionsstillstand. Die Aktivität beträgt noch 44%.

Verhältnis Enzymprotein : Bernsteinsäureanhydrid = 3 : 1. Der Ansatz wird anschliessend mit 1 M Zitronensäure auf pH 5,5 eingestellt; bleibt klar.

Anschliessend erfolgt Dialyse gegen 0,04 M Na-Citrat pH 5,5 enthaltend 0,01% Na-Azid. Die auftretende leichte Trübung wird abzentrifugiert. Aktivität: 33%. Lyophilisation nach Zusatz von 1,2 g Saccharose und 1,8 g Mannit. Lyophilisat: Ausbeute 3,7 g, spez. Akt. 0,178 U/mg; kein Aktivitätsverlust.

Stabilität: 3 Wo. + 4°C = 100% } der Ausgangs-
3 Wo. + 35°C = 85% } aktivität.

Beispiel 14

1 ml Uricase in Glycerin ≙ 20 mg Enzymprotein werden in 24 ml Wasser gegeben und im Eisbad unter Rühren mit 25 mg GANTREZ AN 179 (Copolymer aus Methylvinyläther und Maleinsäureanhydrid) versetzt. Der pH-Wert wird mit 0,1 n NaOH am Titrator bei 9,8 gehalten, dann werden nochmals 25 ml Wasser zugegeben.

Nach Reaktionsstillstand wird mit 1 M Citronensäure auf pH 5,8 eingestellt. Der Ansatz bleibt klar. Es sind noch 60% der Ausgangsaktivität vorhanden. Stabilität: die Aktivität in der Lösung sinkt nach 16 Std. auf 33%, nach 2 Tagen auf 17% und nach 4 Tagen auf 12% des Ausgangswertes ab.

Beispiel 15

Beispiel 14 wird wiederholt, jedoch mit nur 6 mg GANTREZ AN 179. 16 Std. nach pH-Rückstellung auf 5,8 sind noch 48%, nach 3 Tagen noch 38% der Ausgangsaktivität vorhanden. Nach 16 Std. ist die Trübung zu erkennen.

Beispiel 16

6 g Dextran T 40 werden in 40 ml Wasser gelöst und im Eisbad auf ± 0 °C gekühlt. Dann werden 600 mg Cyanurchlorid (2,4,6-Trichlor-1,3,5-triazin) in 10 ml tiefgekühltem Dimethylformamid gelöst und zum Ansatz gegeben. Der pH-Wert wird durch Zusatz von 0,5 n NaOH auf 5,0 eingestellt und gehalten.

Nach Beendigung der Reaktion (keine pH-Änderung mehr) wird das aktivierte Dextran durch dreimalige Umfällung mit einem jeweils 2fachen Volumenüberschuss an tiefgekühltem Aceton gereinigt. Der Aceton-Niederschlag wird jeweils mit Eiswasser gelöst.

Das gereinigte Dextran kann nun entweder als Lösung oder als Lyophilisat zur Proteinfixierung eingesetzt werden.

Zur Fixierung der Uricase werden 1 g Enzymprotein (als Dialysat) in 0,025 M $NaHCO_3/Na_2CO_3$-Puffer, pH 10,2 und das aktivierte Dextran (wässrige Lösung) zusammengegeben und mit Wasser auf 300 ml aufgefüllt.

Anschliessend wird unter Rühren 16 Stunden bei Raumtemperatur inkubiert.

Kontrolle der Fixierung und Lyophilisation:

Der Ansatz wird mit 1 M Citronensäure auf pH 5,5 bis 5,8 eingestellt und 3 Stunden bei Raumtemperatur stehen gelassen.

Eine Opaleszenz bzw. Trübung darf dabei nicht auftreten. Die klare Lösung, die das fixierte Enzym enthält, wird nach Zusatz von 6 g Saccharose und 9 g Mannit auf pH 9,0 eingestellt, mit 750 mg Ammoniumcarbaminat versetzt und lyophilisiert. Die Aktivitätsausbeute beträgt 75 bis 80% bezogen auf die eingesetzte Aktivität. 3 Wochen Lagerung bei 35 °C ergab eine Verringerung der Aktivität um ca. 30%.

**Patentansprüche für die Vertragsstaaten BE · CH · DE · FR · GB · IT · LI · LU · NL · SE**

1. Leber-uricase, dadurch gekennzeichnet, dass sie kovalent an ein wasserlösliches Polysaccharid, Polysäureanhydrid, Polyvinylpyrrolidon oder Säureanhydrid als Trägersubstanz gebunden vorliegt und noch bei pH-Werten unter 6 löslich ist.

2. Leber-Uricase nach Anspruch 1, dadurch gekennzeichnet, dass sie bei pH-Werten zwischen 1 und 11 löslich ist.

3. Leber-Uricase nach Anspruch 1, dadurch gekennzeichnet, dass das lösliche Polysaccharid ein Dextran ist.

4. Leber-Uricase nach Anspruch 1, dadurch gekennzeichnet, dass das lösliche Polysaccharid lösliche Stärke, Zucker oder ein synthetisches Zuckerpolymer ist.

5. Verfahren zur Herstellung einer Leber-Uricase nach den Ansprüchen 1 bis 4, dadurch gekennzeichnet, dass man in an sich bekannter Weise die Uricase in wässriger Lösung mit dem gelösten Trägermaterial, welches wenigstens eine in wässriger Lösung mit $NH_2$-Gruppen reaktionsfähige Gruppe enthält, bei pH 9 bis 11 umsetzt.

6. Verfahren nach Anspruch 5, dadurch gekennzeichnet, dass man ein mit Bromcyan oder Cyanurchlorid aktiviertes Polysaccharid als Trägermaterial verwendet.

7. Verfahren nach Anspruch 5 oder 6, dadurch gekennzeichnet, dass man die erhaltene Lösung der trägergebundenen Uricase mit Saccharose und/oder Mannit versetzt lyophilisiert.

8. Verwendung einer Uricase nach Anspruch 1 bis 7 zur Bestimmung der Harnsäure.

**Patentansprüche für den Vertragsstaat AT**

1. Verfahren zur Herstellung einer bei pH-Werten unter 6 noch löslichen Leber-Uricase, dadurch gekennzeichnet, dass man die Leber-Uricase kovalent an ein wasserlösliches Polysaccharid, Polysäureanhydrid, Polyvinylpyrrolidon oder Säureanhydrid als Trägersubstanz bindet.

2. Verfahren nach Anspruch 1, dadurch gekennzeichnet, dass man als Trägersubstanz Dextran verwendet.

3. Leber-Uricase nach Anspruch 1, dadurch gekennzeichnet, dass man als lösliches Polysaccharid lösliche Stärke, Zucker oder ein synthetisches Zuckerpolymer verwendet.

4. Verfahren nach einem der Ansprüche 1 bis 4, dadurch gekennzeichnet, dass man in an sich bekannter Weise die Uricase in wässriger Lösung mit dem gelösten Trägermaterial, welches wenigstens eine in wässriger Lösung mit $NH_2$-Gruppen reaktionsfähige Gruppe enthält, bei pH 9 bis 11 umsetzt.

5. Verfahren nach Anspruch 4, dadurch gekennzeichnet, dass man ein mit Bromcyan oder Cyanurchlorid aktiviertes Polysaccharid als Trägermaterial verwendet.

6. Verfahren nach Anspruch 4 oder 5, dadurch gekennzeichnet, dass man die erhaltene Lösung der trägergebundenen Uricase mit Saccharose und/oder Mannit versetzt lyophilisiert.

7. Verwendung einer nach den Ansprüchen 1 bis 6 hergestellten Uricase zur Bestimmung der Harnsäure.

**Claims for the Contracting States BE · CH · LI · DE · FR · GB · IT · LU · NL · SE**

1. Liver uricase, characterised in that it is present covalently bound to a water-soluble polysaccharide, polyacid anhydride, polyvinylpyrrolidone

or acid anhydride as carrier substance and is still soluble at pH values below 6.

2. Liver uricase according to claim 1, characterised in that it is soluble at pH values between 1 and 11.

3. Liver uricase according to claim 1, characterised in that the soluble polysaccharide is a dextran.

4. Liver uricase according to claim 1, characterised in that the soluble polysaccharide is soluble starch, sugar or a synthetic sugar polymer.

5. Process for the preparation of a liver uricase according to claims 1 to 4, characterised in that one reacts the liver uricase in per se known manner in aqueous solution at pH 9 to 11 with the dissolved carrier material, which contains at least one group capable of reaction with $NH_2$ groups in aqueous solution.

6. Process according to claim 5, characterised in that one uses a polysaccharide activated with cyanogen bromide or cyanuric chloride as carrier material.

7. Process according to claim 5 or 6, characterised in that one lyophilises the solution of carrier-bound uricase obtained, mixed with saccharose and/or mannitol.

8. Use of a uricase according to claims 1 to 7 for the determination of uric acid.

**Claims for the Contracting State AT**

1. Process for the preparation of a liver uricase still soluble at pH values below 6, characterised in that one binds the liver uricase covalently to a water-soluble polysaccharide, polyacid anhydride, polyvinylpyrrolidone or acid anhydride as carrier substance.

2. Process according to claim 1, characterised in that one uses dextran as carrier substance.

3. Process according to claim 1, characterised in that, as soluble polysaccharide, one uses soluble starch, sugar or a synthetic sugar polymer.

4. Process according to one of claims 1 to 4, characterised in that, in per se known manner, one reacts the uricase in aqueous solution with the dissolved carrier material, which contains at least one group capable of reaction in aqueous solution with $NH_2$ groups, at pH 9 to 11.

5. Process according to claim 4, characterised in that, as carrier material, one uses a polysaccharide activated with cyanogen bromide or cyanuric chloride.

6. Process according to claim 4 or 5, characterised in that one lyophilises the solution obtained of the carrier-bound uricase mixed with saccharose and/or mannitol.

7. Use of a uricase prepared according to one of claims 1 to 6 for the determination of uric acid.

**Revendications pour les Etats contractants BE · CH · LI · DE · FR · GB · IT · LU · NL · SE**

1. Uricase hépatique, caractérisée en ce qu'elle est présente sous forme liée par convalence à un polysaccharide, à un polyanhydride d'acide, à la polyvinylpyrrolidone ou à un anhydride d'acide solubles dans l'eau comme substance de support, et en ce qu'elle est encore soluble pour des pH inférieurs à 6.

2. Uricase hépatique selon la revendication 1, caractérisée en ce qu'elle est soluble à des pH entre 1 et 11.

3. Uricase hépatique selon la revendication 1, caractérisée en ce que le polysaccharide soluble est un dextrane.

4. Uricase hépatique selon la revendication 1, caractérisée en ce que le polysaccharide soluble est un amidon soluble, un sucre ou un polymère de sucre synthétique.

5. Procédé de préparation d'une uricase hépatique selon les revendications 1 à 4, caractérisé en ce qu'on fait réagir l'uricase d'une manière connue en soi en solution aqueuse avec la matière de support dissoute, laquelle contient au moins un groupe capable de réagir avec des groupes $NH_2$ en solution aqueuse, à un pH de 9 à 11.

6. Procédé selon la revendication 5, caractérisé en ce qu'on utilise comme matière de support un polysaccharide activé par du bromure de cyanogène ou du chlorure de cyanuryle.

7. Procédé selon la revendication 5 ou 6, caractérisé en ce qu'on lyophilise la solution obtenue de l'uricase liée à un support additionnée de saccharose et/ou de mannitol.

8. Utilisation d'une uricase selon les revendications 1 à 7 pour le dosage de l'acide urique.

**Revendications pour l'état contractant: AT**

1. Procédé pour la préparation d'une uricase hépatique encore soluble à des valeurs de pH inférieures à 6, caractérisé en ce que l'on fixe l'uricase hépatique de façon covalente à un polysaccharide, un polyanhydride d'acide, une polyvinylpyrrolidone ou un anhydride d'acide, soluble dans l'eau, comme substance de support.

2. Procédé selon la revendication 1, caractérisé en ce qu'on utilise le dextrane comme substance de support.

3. Uricase hépatique selon la revendication 1, caractérisée en ce que l'on utilise, en tant que polysaccharide soluble, un amidon soluble, un sucre ou un polymère de sucre synthétique.

4. Procédé selon l'une des revendications 1 à 3, caractérisé en ce qu'on fait réagir l'uricase d'une manière connue en soi en solution aqueuse avec la matière de support dissoute, laquelle contient au moins un groupe capable de reagir avec des groupes $NH_2$ en solution aqueuse, à un pH de 9 à 11.

5. Procédé selon la revendication 4, caractérisé en ce qu'on utilise comme matière de support un polysaccharide activé par du bromure de cyanogène ou du chlorure de cyanuryle.

6. Procédé selon la revendication 4 ou 5, caractérisé en ce qu'on lyophilise la solution obtenue de l'uricase liée à un support additionnée de saccharose et/ou de mannitol.

7. Utilisation d'une uricase préparée selon l'une des revendications 1 à 6 pour le dosage de l'acide urique.